Europäisches Patentamt

European Patent Office

Office européen des brevets

(11) Publication number: **0 099 734**
**B1**

(12) **EUROPEAN PATENT SPECIFICATION**

(45) Date of publication of patent specification: **08.10.86**

(51) Int. Cl.⁴: **A 61 N 1/42**

(21) Application number: **83304103.1**

(22) Date of filing: **14.07.83**

(54) Magnetotherapeutic impulse device.

(30) Priority: **20.07.82 CS 5549/82**

(43) Date of publication of application:
**01.02.84 Bulletin 84/05**

(45) Publication of the grant of the patent:
**08.10.86 Bulletin 86/41**

(84) Designated Contracting States:
**AT BE CH DE FR GB IT LI NL SE**

(56) References cited:
**EP-A-0 039 206**
**EP-A-0 042 889**
**DE-A-2 822 285**
**FR-A- 951 906**
**FR-A-2 370 483**

**Prospectus "PAMATRON" Medizinische Geräte
K.G.; Buchen/ODW., FRG**

(73) Proprietor: **Tesla, Koncernovy Podnik
No. 186 Podebradska
Praha 9 (CS)**

(72) Inventor: **Mudr, Jiri Jerabek
No. 421 Nad upadem
Praha 4 (CS)**

(74) Representative: **Matthews, Howard Nicholas
et al
MATTHEWS HADDAN & CO Haddan House
33 Elmfield Road
Bromley Kent BR1 1SU (GB)**

Courier Press, Leamington Spa, England.

## Description

The invention relates to a circuit arrangement for magnetotherapeutic impulse device, particularly adapted for treatment of inflammatory and degenerative illness.

Known magnetotherapeutic impulse devices of this kind are either based on the discharge of a capacitor to the winding of an electromagnet or on a connection of the winding of an electromagnet in a multivibrator circuit, on closing a current circuit of a thyristor coil which is closed by a multivibrator at a variable frequency, possibly by closing of the current of a coil by a thyristor controlled by a divided and adjusted frequency of the network or by closing direct current flowing to a coil of an electromagnet by a mechanical motor driven switch. A circuit arrangement having the features according to the pre-characterising part of claim 1 is known from EP—A—0042889.

A drawback of existing devices is mostly the impossibility of controlling the power supplied to coils, there is a lack of accurate adjustment of the repeating frequency of the magnetic field, no solution is known, having the possibility of an automatic control of changing the repeating frequency. At all devices there is a possibility to connect in the circuit a small number, up to four coils. Solely some solutions secure a synchronization of the closing of the current to coils with the phase of the supplied voltage and thus with the uniformity of impulses of the magnetic field. The majority of known solutions uses mechanical switching, increasing thereby the size and reducing its reliability.

It is an object of this invention to eliminate or at least to reduce these drawbacks.

According to the invention there is provided a circuit arrangement for a magnetotherapeutic impulse device having

a plurality of electromagnetic coils which are connected in series with a power switching element and with a voltage source;

means for controlling the excitation frequency of the electromagnet coils; and

means for controlling the excitation power applied to the electromagnet coils, whereby the excitation frequency and power applied to one or more of the electromagnetic coils are adjustable, characterised in that the output of a circuit for sinewave adjustment of the rectangular shape voltage, which circuit is connected to the voltage source, is connected to the clock input of an electronic changeover switch and also to the clock input of a control block for a multiplexer, that the outputs of the electronic changeover switch are connected to the inputs of phasing elements, the outputs of which are connected to the control inputs of the power switching elements, that the outputs of the electronic changeover switch, starting with its second output, are furthermore connected to the inputs of a multiplexer, that the control block for the multiplexer is connected to the control input of the multiplexer, and that the output of the multiplexer is connected to a zero input of the electronic changeover switch.

A block indicating the adjusted frequency can be connected to the output of the control block for the multiplexer.

A timing switch can be connected between the output of the circuit for voltage adjustment and the clock inputs of the electronic changeover switch and of the control block for the multiplexer.

Circuits indicating the changeover can be connected in parallel to phasing elements.

Separating elements can be connected between the control inputs of the power switching elements and the outputs of the phasing elements.

Circuits indicating the excitation of a magnetic field can be connected in parallel to the electromagnetic coils.

A n-pole changeover switch, connected by opposite contacts to the circuit of a recording unit, can be connected in parallel to the electromagnetic coils.

Advantages of the arrangement according to this invention are primarily the possibility of a control of power for individual electromagnet coils and the possibility of a continuous, automatic and manual control of the change of the repeating frequency of the generated magnetic field, securing the possibility of an accurate dosing of the magnetic field at a medical application of the arrangement. Due to the possibility of a simultaneous connection of a large number of electromagnet coils there is a possibility of application of the magnetic field for instance to a larger number of joints to be treated, to the whole spine of the like. Due to the mentioned interconnection, the synchronism of switching of power switching elements with the phase of the voltage supplied by the voltage source is secured, what secures the uniformity of impulses of the magnetic field excited by electromagnet coils. By application of electronic elements for contactless switching and by digital control a high reliability of operation of the described arrangement and a minimum consumption of power are obtained.

The circuit arrangement of the magnetotherapeutic impulse device according to this invention will be in the following described in an examplary embodiment with reference to the accompanying drawings, where

FIGURE 1 shows a block wiring diagram of the fundamental interconnection of the magnetotherapeutic impulse device;

FIGURE 2 shows an extended block wiring diagram as in Figure 1 with application of additional circuits and elements;

FIGURE 3 shows a specific examplary interconnection of the magnetotherapeutic impulse device.

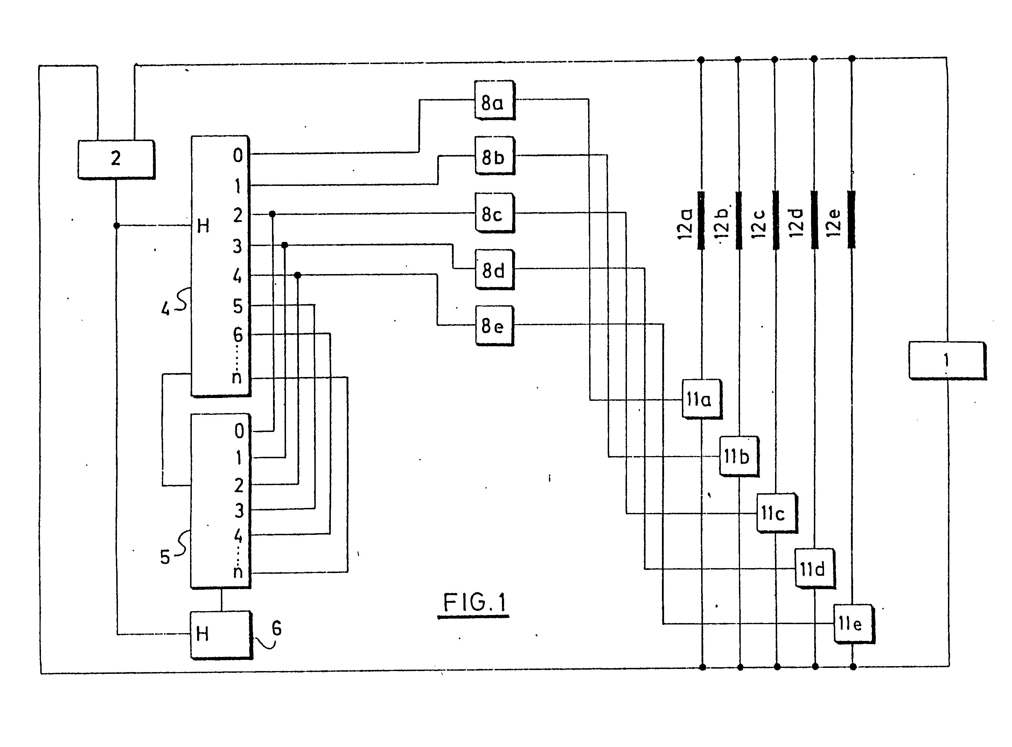

According to Figure 1, showing the fundamental interconnection of the magnetotherapeutic impulse device, a circuit 2 for voltage adjustment, connected to a voltage source 1, is connected by its output both to a clock input of an electronic changeover switch 4 and also to a clock input of a control block 6 for the control of a multiplexer 5. The outputs of the electronic changeover switch 4 are connected to inputs of phasing elements 8a to 8e, the outputs of which are connected to control inputs of power switching elements 11a to 11e, each of which is connected in series with electromagnet coils 12a to 12e. Each of said series is connected to the voltage source 1. Furthermore, starting with the second output, the outputs of changeover switch 4 are connected to inputs of the multiplexer 5. The control block 6 for the multiplexer 5 is connected with the control input of the multiplexer 5. The output of the multiplexer is connected to a zero input of the electronic changeover switch 4.

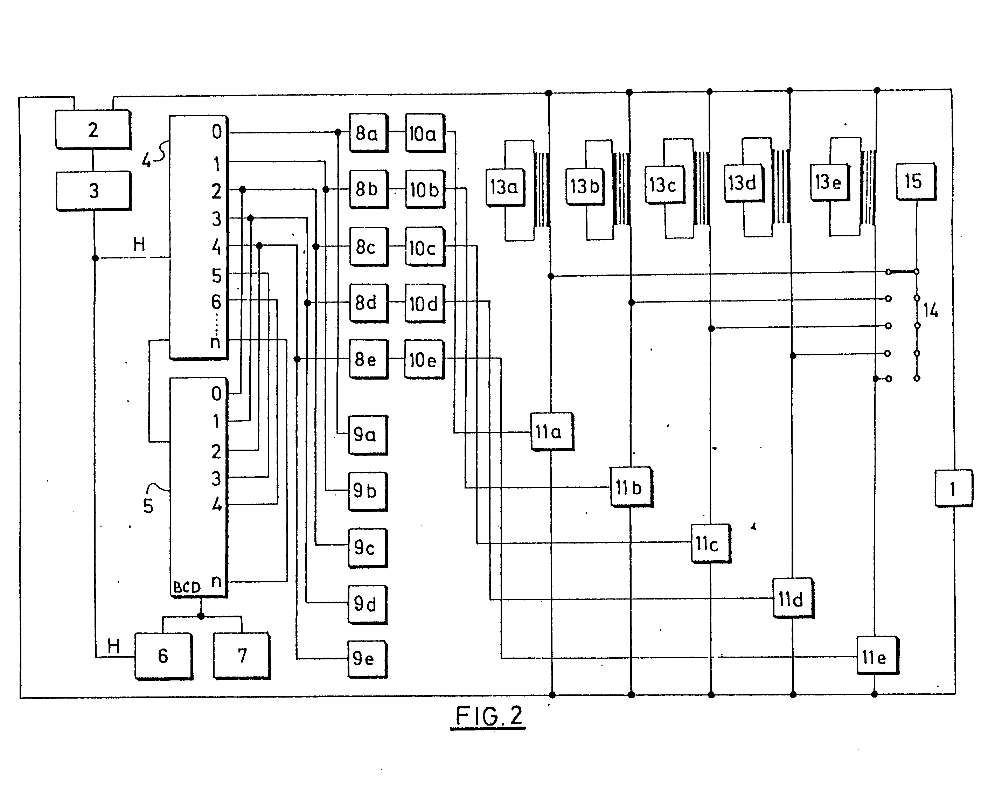

In order to increase the effect, the fundamental circuit arrangement of the magnetotherapeutic impulse device is completed by following circuit arrangements as shown in Figure 2.

A block 7 indicating the adjusted frequency is connected to the output of the control block 6 for control of the multiplexer 5.

A timing switch 3 is connected between the output of circuit 2 for voltage adjustment and between clock inputs of the electronic switch 4 and the control block 6 for the control of the multiplexer.

Circuits 9a to 9e indicating the switching over are connected in parallel to inputs of phasing elements 8a to 8e.

Separating elements 10a to 10e are connected between the control inputs of power switching elements 11a to 11e and outputs of phasing elements 8a to 8e.

Indicating circuits 13a to 13e are connected in parallel to electromagnet coils 12a to 12e.

A simple five pole changeover switch 14 is connected by opposite contacts to the circuit 15 of the recording unit in parallel to electromagnet coils 12a to 12e.

In operation of the circuit arrangement of the magnetotherapeutic impulse device of Figure 1 for example, the circuit 2 for voltage adjustment connected to the voltage source 1 adjusts the supplied sine voltage to the required magnitude and changes the shape of the supplied voltage to a voltage of rectangular shape. The thus adjusted voltage is supplied both to the clock input of the electronic changeover switch 4 and to the clock input of the control block 6 for control of the multiplexer. At individual outputs of the electronic changeover switch 4 a consecutive changeover of logic levels takes place, whereby the speed of the chnageover depends on the frequency of the voltage supplied to the clock input of the electronic changeover switch 4. The outputs $0$ to $n$ of the electronic changeover switch 4 are connected both with inputs of phasing elements 8a to 8e, each independently securing a time delay of the signals on their outputs with respect to the input signal. Thus, also the phase shift of closing of power switching elements 11a to 11e with respect to the phase of voltage supplied from the voltage source 1, is independently secured. The series connection of coils 12a to 12e of the electromagnets with the power switching elements 11a to 11e and with the voltage source 1 secures the possibility of control of power to individual electromagnetic coils 12a to 12e and thus the possibility of control of the magnetic field intensity. Furthermore, starting with the second output, the outputs of the electronic changeover switch 4 are connected to inputs $0$ to $n$ of the multiplexer 5, the output of which is connected to the zeroing input of the electronic changeover switch 4. The control of the multiplexer 5 is achieved by means of the control block 6 for the control of the multiplexer. This control block 6 for the control of the multiplexer which is connected by its clock input to the circuit 2 for adjustment of the voltage and by its output to the control input of the multiplexer 5 secures by means of the multiplexer 5 a transmission of logic levels from the outputs of the electronic changeover switch 4 to the zero input of the electronic changeover switch 4 and thus, according to an adjustment of the control block 6 for control of the multiplexer provides the possibility of reduction or prolongation of the changeover cycle of the electronic changeover switch 4. By this kind of interconnection of the magnetotherapeutic impulse device the synchronism of excitation of the magnetic field by electromagnet coils 12a to 12e with voltage supplied from the voltage source 1 is secured, as well as the possibility of adjustment of power to individual electromagnet coils 12a to 12e. Finally, via the connection of the electronic changeover switch 4 to the multiplexer 5 and via its control by the control block 6 for the multiplexer the changeover cycle of the electromagnetic changeover switch 4 can be shortened and thus the repeating frequency of the magnetic field excited by electromagnet coils 12a to 12e can be changed.

In the fundamental interconnection with the additional circuits as shown in Figure 2 the following improvements of the functioning are achieved.

Information about the adjusted frequency is secured by connection of the block 7 for indication of the adjusted frequency to the output of the control block 6 for control of the multiplexer.

The adjustment of the time, during which the patient is exposed to treatment, is secured by introduction of the timing switch 3 between the outputs of circuit 2 for adjustment of the voltage and between the clock inputs of the electronic changeover switch 4 and of the block 6 for the control of the multiplexer.

Information about the operation of the logic part of the device is secured by the parallel connection of the circuit 9a to 9e for indication of changeover to inputs of phasing elements 8a to 8e.

The separation of logic parts of the device from power parts by introduction of separating elements 10a to 10e between control inputs of power switching elements 11a to 11e and outputs of phasing elements 8a to 8e.

Information about the excited magnetic field and simultaneously about a possible failure of power switching elements 11a to 11e are secured by a parallel connection of indication elements 13a to 13e to electromagnet coils 12a to 12e.

Information about the shape and amplitude of the voltage on individual electromagnet coils 12a to 12e is secured by parallel connection of a simple n-pole changeover switch 14 to electromagnet coils 12a to 12e and by connection of its opposite, mutually interconnected contacts to circuits 15 of a recording unit.

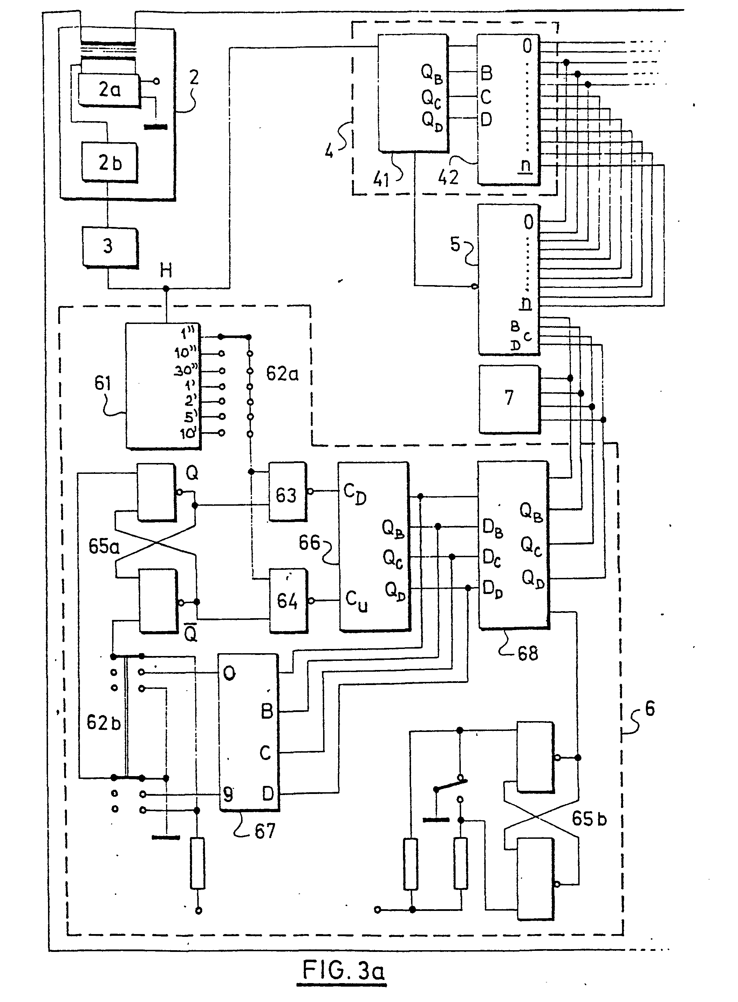

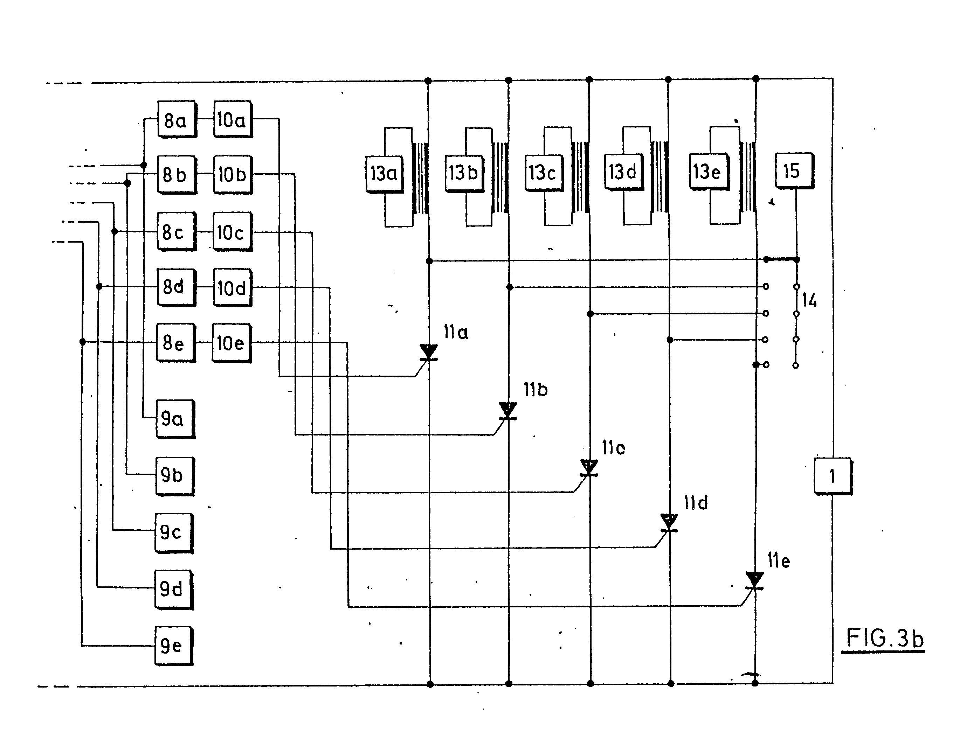

An example of a practical interconnection of a magnetotherapeutic impulse device according to this invention is shown in combined Figure 3a and 3b. The network voltage from the voltage source 1 is supplied to the input of circuit 2 for adjustment of voltage comprising a main transformer, from the secondary winding of which a stabilizer 2a of DC voltage is supplied for feeding light current parts of the device. A voltage sample for the pulse shaper 2b changing the sine voltage to a rectangular shape is furthermore tapped off from the secondary winding. The thus adjusted voltage is supplied to the input of the timing switch 3, by way of which it passes for a predetermined time to the output and therefrom both to the clock input of the electronic changeover switch 4 and to the clock input of block 6 for control of the multiplexer. The electronic changeover switch 4 comprises a counter 41 and a 1 of n converter 42, which by its BCD inputs is connected to outputs QB, QC, QD of the counter 41. The supplied clock frequency is coded by the counter 41 to a BCD code and the 1 of n converter 42 switches over the logic levels of its outputs in a rhythmus of the supplied clock frequency. The outputs of the electronic changeover switch 4 are connected to the inputs of the phasing elements 8a to 8e, to which inputs of circuits 9a to 9e for indication of the changeover are connected in parallel, which inform for instance optically about logic levels of outputs of the electronic changeover switch 4. The phasing elements 8a to 8e secure the phase displacement of the signal on their outputs with respect to the phase of the signal on their inputs and thus also with respect to the phase of the AC voltage supplied from the voltage source 1. The outputs of the phasing elements 8a to 8e are connected with inputs of separating elements 10a to 10e, the outputs of which are connected to control inputs of power switching elements 11a to 11e, (e.g., thyristors as shown). The separating elements 10a to 10e (e.g., opto-isolators) secure the transmission of the signal from the phasing elements 8a to 8e to control inputs of power switching elements 11a to 11e and separate galvanically the control electronics from the power part of the arrangement. By supply of the signal to control inputs of power switching elements 11a to 11e, these elements are brought to conductive condition dependent on the firing angle delaying the ignition of the thyristors. Due to their series connection with electromagnet coils 12a to 12e and with the voltage source 1, electric current phases through the circuit which excites the magnetic field. The indicating circuit 13a to 13e connected in parallel to coils 12a to 12e inform optically by a flash about the excitation of the magnetic field and simultaneously serve as indicator in case of a possible failure of power switching elements 11a to 11e. In case of said failure they are illuminated continually at a continually passing current and thus indicate a continually excited alternating magnetic field. According to the position of a simple n-pole changeover switch 14 connected to electromagnet coils 12a to 12e and by its opposite contacts to the circuit 15 of the recording unit, the attendants obtain information about the amplitude and shape of the voltage impulse at individual electromagnet coils 12a to 12e. The clock frequency supplied from the output of the timing switch 3 to the clock input of block 6 for control of the multiplexer is supplied to the input of the separating section 61 and therefrom voltage of rectangular shape of a frequency time interval 1'', 10'', 30'', 1', 2', 5' and 10' is taken off from individual outputs and supplied to contacts of a simple multipole changeover switch 62a, the opposite contacts of which are mutually interconnected. By means of multipole changeover switch 62a the voltage of a chosen frequency is supplied to first inputs of positive twin input gates 63 and 64, to the second inputs of which the control circuit 65a is connected by its outputs Q and Q̄. The outputs of positive twin input gates 63 and 64 are connected to inputs CD and CU of a reversible counter 66. The switching over of the control circuit 65a is accomplished by means of the changeover switch 62b which secures in both extreme positions that on outputs Q and Q̄ of the control circuit 65a will secure transmission of the signal by the positive twin stage gate 63 and 64 either to the input CD or CU of the reversible counter 66, whereby a counting ahead or rearwards is secured. In the middle position of the changeover switch 62b, the automatic changeover of conditions of outputs Q and Q̄ of the control circuit 65a by means of connected outputs 0 and 9 of the 1 of n converter 67 to middle contacts of the changeover switch 62b is secured. The BCD inputs of the 1 of n converter 67 is connected to the outputs QB, QC, QD of the reversible counter 66, whereby for instance in case of counting ahead after reaching a binary coded number $n-9$ the automatic changeover of the reversible counter 66 for the operation of counting rearwards is secured, furthermore after reaching the binary number 0 for changeover of counting ahead is secured and the whole cycle is constantly repeated. The outputs QB, QC, QD of the reversible counter 66 are furthermore connected to inputs DB, DC, DD of the memory 68. By means of the changeover switch 62a and the circuit 65 for locking the memory it is possible to prevent transmission of a binary coded number from the input to the output and to maintain the thus chosen number on the output of the memory 68 without regard to the actual condition of outputs QB, QC, QD of the reversible counter 66. The outputs QB, QC, QD of the memory 68 are connected to the inputs of the block 7 for indication of the adjusted frequency and to the BCD inputs of the multiplexer 5.

The outputs of the electronic changeover switch 4, starting with the second output are connected to inputs 0 to n of the multiplexer 5. A further output of the multiplexer 5 is connected to the zero input of the electronic changeover switch 4. The functioning of the thus interconnected multiplexer 5 consists in that the logic levels supplied from the outputs of the electronic changeover switch 4 are transmitted to the output of the multiplexer solely from that output, the number of which corresponds to the binary coded number on the BCD input of the multiplexer 5. The logic level H, supplied from the output to the zero input of the electronic changeover switch 4, or 41 respectively, is thus eliminated. This effects a return of the changeover of the electronic changeover switch 4 to the output 0.

The dependence of the fact which input of the multiplexer 5 transmits, a shortening or prolongation of the counting cycle of the counter 41 is secured and thus via 1 of n converter 42, phasing elements 8a to 8e, separating elements 10a to 10e and the control inputs of power switching elements 11a to 11e an increase or reduction of the repeating frequency of the magnetic field excited by the electromagnet coils 12a to 12e. The block 7 for indication of the adjusted frequency informs about the just adjusted repeating frequency of the excited magnetic field.

This interconnection of the magnetotherapeutic impulse device secures the possibility of a selection of the repeating frequency of the magnetic field, which frequency is determined by dividing the frequency of the voltage of the voltage source 1 by a whole number however at least by 2. It is possible to select either a single frequency by closing the memory 68 by the changeover switch 62a via the circuit 65b for locking the memory. It is furthermore possible to select, based on an adjustment of changeover switches 62a, 62b at intervals determined by the separation section 61, an automatic increase or an automatic lowering of the repeating frequency of the excited magnetic field or an automatic increase and a following decrease or vice versa. By means of adjustment of the phasing elements 8a to 8e it is possible to secure the phase displacement of the closing of power switching elements with respect to the phase of supplied voltage from the voltage source 1 and thus the adjustment of power supplied to individual electromagnet coils 12a to 12e. By adjustment of the timing switch 3 a standstill of the arrangement after a predetermined time is secured. The circuit 7 for indication of the adjusted frequency informs about the repeating frequency of the excited magnetic field. The circuits 9a to 9e for indicating the changeover inform about a correct functioning of the electronic changeover switch 4. The indicating circuit 13a to 13e are informing about the presence of the excited magnetic field. By means of a simple n-pole changeover switch 14 and by the circuit 15 of the recording unit it is possible to check the amplitude and shape of voltage impulses on electromagnet coils 12a to 12e.

**Claims**

1. Circuit arrangement for magnetotherapeutic impulse device ahving a plurality of electromagnetic coils (12a — 12e), which are connected in series with respective power switching elements (11a — 11e) and with a voltage source (1), means (4, 5 and 6) for controlling the excitation frequency of the electromagnetic coils; and means (8a — 8e) for controlling the excitation power applied to the electromagnetic coils, whereby the excitation frequency and power applied to one or more of the electromagnetic coils are adjustable, characterised in that the output of a circuit (2) for voltage adjustment and for changing the sinewave from the voltage source to rectangular shape, which circuit is connected to the voltage source (1), is connected to the clock input of an electronic changeover switch (4) and to the clock input of a control block (6) for a multiplexer (5), that outputs (0 to n) of the electronic changeover switch (4) are connected the to inputs of phasing elements (8a to 8e), the outputs of which are connected to control inputs of the power switching elements (11a to 11e), that the outputs of the electronic changeover switch (4) starting with its second output, are furthermore connected to the inputs (0 and n) of the multiplexer (5), that the control block (6) for the multiplexer is connected to the control input of the multiplexer (5), and that the output of the multiplexer is connected to the zero input of the electronic changeover switch (4).

2. Circuit arrangement as claimed in Claim 1, characterised in that a block (7) for the indication of the adjusted frequency is connected to the output of the control block (6) for the multiplexer.

3. Circuit arrangement as claimed in Claim 1 or 2, characterised in that a timing switch (3) is connected between the output of the circuit (2) for voltage adjustment and the clock inputs of the electronic changeover switch (4) and of the control block (6) for the multiplexer.

4. Circuit arrangement as claimed in Claim 1, 2 or 3, characterised in that circuits (9a to 9e) for the indication of the changeover are connected in parallel to the inputs of the phasing elements (8a to 8e).

5. Circuit arrangement as claimed in one of Claims 1 to 4 characterised in that separating elements (10a to 10n) are connected between the control inputs of the power switching elements (11a to 11e) and the outputs of the phasing elements (8a to 8e).

6. Circuit arrangement as claimed in one of Claims 1 to 4, characterised in that circuits (13a to 13n) indicating the excitation of a magnetic field are connected to the electromagnetic coils (12a to 12e).

7. Circuit arrangement as claimed in one of Claims 1 to 5, characterised in that an n-pole changeover switch (14), connected by opposite contacts to a circuit (15) of a recording unit, is connected in parallel to the electromagnetic coils (12a to 12n).

## Patentansprüche

1. Schaltungsanordnung für magnetotherapeutische Impulseinrichtung, mit einer Vielzahl von elektromagnetischen Spulen (12a—12e), die jeweils mit Lastschaltelementen (11a—11e) verbunden sind, und mit einer Spannungsquelle (1), Mitteln (4—6) zur Steuerung der Erregerfrequenz der elektromagnetischen Spulen; und eine Vorrichtung (8a—8e) zur Steuerung der den elektromagnetischen Spulen zugeführten Erregerleistung, wobei die einer oder mehreren der elektromagnetischen Spulen zugeführte Erregerfrequenz und Leistung steuerbar sind, dadurch gekennzeichnet, dass der Ausgang eines Stromkreises (2) für eine Spannungsanpassung und zur Änderung der Sinuswelle von der Leistungsquelle auf eine Viereckform, welcher Stromkreis mit der Spannungsquelle (1) verbunden ist, am Zeitelement eines elektronischen Umschalters (4) und am Zeitelement eines Steurblocks (6) für einen Vielfachschalter verbunden ist, dass die Ausgänge (0 bis n) des elektronischen Umschalters (4) mit den Eingängen von Phasenlageelementen (8a—8e) verbunden sind, deren Ausgänge an Steuereingängen von Leistungsschaltelementen (11a—11e) angeschlossen sind, dass die Ausgänge des elektronischen Umschalters (4) von seinem zweiten Ausgang an ferner mit den Eingängen (0 bis n) des Vielfachschalters (5) verbunden sind, dass der Steuerblock (6) für die Vielfachschaltung am Steuereingang des Vielfachschalters (5) angeschlossen ist, und dass dessen Ausgang mit dem Nulleingang des elektronischen Umschalters (4) verbunden ist.

2. Schaltungsanordnung nach Anspruch 1, dadurch gekennzeichnet, dass ein Block (7), zur Anzeige der eingestellten Frequenz, am Ausgang des Steuerblockes (6) für den Vielfachschalter angeschlossen ist.

3. Schaltungsanordnung nach Anspruch 1 oder 2, dadurch gekennzeichnet, dass ein Zeitschalter (3) zwischen dem Ausgang des Stromkreises (2) für eine Spannungseinstellung un den Zeiteingängen des elektronischen Umschalters (4) und des Steuerblocks (6) für die Vielfachschaltung angeschlossen ist.

4. Schaltungsanordnung nach Anspruch 1, 2 oder 3, dadurch gekennzeichnet, dass die Stromkreise (9a—9e) zur Anzeige der Umschaltung mit den Eingängen der Phasenlageelemente (8a—8e) parallelverbunden sind.

5. Schaltungsanordnung nach einem der Ansprüche 1 bis 4, dadurch gekennzeichnet, dass Trennelements (10a—10n) zwischen den Steuereingängen der Lastschaltelemente (11a—11e) und den Ausgängen der Phasenlageelemente (8a—8e) angeschlossen sind.

6. Schaltungsanordnung nach einem der Ansprüche 1 bis 4, dadurch gekennzeichnet, dass Stromkreise (13a—13n) zur Anzeige der Erregung eines Magnetfeldes mit den elektromagnetischen Spulen (12a—12e) verbunden sind.

7. Schaltungsanordnung nach einem der Ansprüche 1 bis 5, dadurch gekennzeichnet, dass ein n-poliger Umschalter (14), der mittels gegenüberliegender Kontakte mit einem Stromkreis (15) eines Aufzeichnungsgerätes verbunden ist, an den elektromagnetischen Spulen (12a—12n) angeschlossen ist.

## Revendications

1. Organisation de circuit pour dispositif de production d'impulsions magnétothérapeutiques comportant un certain nombre de bobines électromagnétiques (12a à 12e), branchées en série avec des éléments de commutation de puissance correspondants (11a à 11e) et avec une source de tension (1), des moyens (4, 5 et 6) destinés à commander la fréquence d'excitation des bobines électromagnétiques; et des moyens (8a à 8e) destinés à commander la puissance d'excitation appliquée aux bobines électromagnétiques, de sorte que la fréquence et la puissance d'excitation appliquées à l'une ou plusieurs des bobines électromagnétiques sont réglables, organisation de circuit caractérisée en ce que la sortie d'un circuit (2) de réglage de tension et de changement de la forme d'onde sinusoïdale de la source de tension en une forme d'onde rectangulaire, ce circuit étant branché à la source de tension (1), est reliée à l'entrée d'horloge d'un commutateur d'inversion électronique (4) et à l'entrée d'horloge d'un bloc de commande (6) d'un multiplexeur (5), en ce que les sorties (0 à n) du commutateur d'inversion électronique (4) sont reliées aux entrées d'éléments de mise en phase (8a à 8e) dont les sorties sont branchées aux entrées de commande des éléments de commutation de puissance (11a à 11e), en ce que les sorties du commutateur d'inversion électronique (4) déclenchées avec sa seconde sortie, sont, en outre, branchées aux entrées (o à n) du multiplexeur (5), en ce que le bloc de commande (6) du multiplexeur est branché à l'entrée de commande du multiplexeur (5), et en ce que la sortie du multiplexeur est branchée à l'entrée zéro du commutateur d'inversion électronique (4).

2. Organisation de circuit selon la revendication 1, caractérisèe en ce qu'un bloc (7) d'indication de la fréquence de réglage est branché à la sortie du bloc de commande (6) du multiplexeur.

3. Organisation de circuit selon l'une quelconque des revendications 1 et 2, caractérisèe en ce qu'un commutateur de synchronisation (3) est branché entre la sortie du circuit (2) de réglage de tension et les entrées d'horloge du commutateur d'inversion électronique (4) et du bloc de commande (6) du multiplexeur.

4. Organisation de circuit selon l'une quelconque des revendications 1 à 3, caractérisèe en ce que des circuits 9a à 9e d'indication d'inversion sont branchés en parallèle sur les entrées des éléments de mise en phase (8a à 8e).

5. Organisation de circuit selon l'une quelconque des revendications 1 à 4, caractérisée en ce que des éléments de séparation (10a à 10n) sont branchés entre les entrées de commande des éléments de commutation de puissance (11a à 11e) et les sorties des éléments de mise en phase (8a à 8e).

6. Organisation de circuit selon l'une quelconque des revendications 1 à 4, caractérisée en ce que des circuits (13a à 13n) d'indication de l'excitation d'un champ magnétique sont branchés aux bobines électromagnétiques (12a à 12e).

7. Organisation de circuit selon l'une quelconque des revendications 1 à 5, caractérisée en ce qu'un commutateur d'inversion à n pôles (14), relié par des contacts opposés à un circuit (15) d'un bloc d'enregistrement, est branché en parallèle sur les bobines électromagnétiques (12a à 12n).

FIG.1

0 099 734

FIG.2

0 099 734

FIG. 3a

FIG. 3b